# EUROPEAN PATENT SPECIFICATION

(11) **EP 3 648 731 B1**
(45) Date of publication and mention of the grant of the patent: **17.02.2021**
(21) Application number: 18732344.9
(22) Date of filing: 21.06.2018
(51) Int. Cl.: A61K 8/02, A61K 8/73, A61K 8/23, A61K 8/365, A61Q 11/00, A61K 8/19, A61K 8/25, A61K 8/24

(54) **ORAL CARE COMPOSITION**
MUNDPFLEGEZUSAMMENSETZUNG
COMPOSITION D'HYGIÈNE BUCCALE

(30) Priority: 07.07.2017 WO PCT/CN2017/092182; 01.08.2017 EP 17184164
(43) Date of publication of application: 13.05.2020
(73) Proprietor: Unilever N.V., 3013 AL Rotterdam (NL); Unilever PLC, London, Greater London EC4Y 0DY (GB)
(72) Inventor: GREEN, Alison, Katharine, Wirral Merseyside CH63 3JW (GB); LI, Xiaoke, Shanghai 200335 (CN); SUN, Yuekui, Shanghai 200335 (CN); XING, Huaiyong, Shanghai 200120 (CN); ZHOU, Huanjun, Shanghai 200335 (CN); WANG, Jinfang, Shanghai 200335 (CN)
(74) Representative: Tansley, Sally Elizabeth
(86) International application number: PCT/EP2018/066525
(87) International publication number: WO 2019/007695

(56) References cited:
- WO-A1-2017/005431
- WO-A1-2017/080687
- WO-A2-2012/031785
- WO-A2-2013/034421

## Description

### Technical Field of the Invention

The present invention relates to oral care compositions such as tooth pastes, powders, gums, mouthwashes and the like. In particular the present invention relates to an oral care composition comprising a composite material that results in reducing tooth sensitivity and/or tooth remineralization. The invention also relates to the use of such compositions for treating tooth hypersensitivity and/or remineralizing and/or whitening of teeth of an individual.

### Background of the Invention

Tooth hypersensitivity is a temporary induced pain sensation that affects up to 20% of the adult population. Hypersensitive teeth may be sensitive to temperature, pressure or chemical action.

The dentin of the tooth generally contains channels, called tubules, which provide for an osmotic flow between the inner pulp region of the tooth and the outer root surfaces. Tooth hypersensitivity may be related to the general increase in exposed root surfaces of teeth as a result of periodontal disease, toothbrush abrasion, or cyclic loading fatigue of the thin enamel near the dentin-enamel junction. When root surfaces are exposed, dentinal tubules are also exposed.

The currently accepted theory for tooth hypersensitivity is the hydrodynamic theory, based on the belief that open exposed dentinal tubules allow fluid flow through the tubules. This flow excites the nerve endings in the dental pulp. Clinical replica of sensitive teeth viewed in a SEM (scanning electron microscopy) reveal varying numbers of open or partially occluded dentinal tubules.

There are different approaches to treat tooth hypersensitivity. One approach is to reduce the excitability of the nerve in a sensitive tooth by using "nerve-depolarising agents" comprising strontium ions, potassium salts such as potassium nitrate, potassium bicarbonate, potassium chloride and the like. These nerve-depolarising agents function by interfering with neural transduction of the pain stimulus to make the nerve less sensitive. Another approach is to use "tubule blocking agents" that fully or partially occlude tubules such as polystyrene beads, apatite, polyacrylic acid, mineral hectorite clay and the like. These tubule blocking agents function by physically blocking the exposed ends of the dentinal tubules, thereby reducing dentinal fluid movement and reducing the irritation associated with the shear stress described by the hydrodynamic theory.

The present inventors have found unexpectedly that an oral care composition comprising a composite material is more efficient for treating tooth hypersensitivity. The composite material comprises a first component material comprising a biomineralization agent and a second component material capable of reacting with phosphate ions to produce a calcium and phosphate *in situ* reaction product that adheres to tooth enamel, dentin or both and that is a reagent for hydroxyapatite formation. Such composite material is found to effectively deposit on tooth surfaces even during routine oral hygiene practices such as tooth brushing. The oral care composition provides excellent tubule blockage efficacy to reduce tooth sensitivity. In addition, such composition can also enhance the tooth remineralization efficacy and/or the deposition of benefit agents on tooth surfaces to further benefit teeth of an individual.

### Additional Information

WO 2008/068149 A (Unilever) discloses an oral care product comprising a first composition comprising an insoluble calcium salt that is not a calcium phosphate salt, a second independent composition comprising a source of phosphate ions, and a means for delivering each of the compositions to the surface of the teeth. The preferred insoluble calcium salt is calcium silicate.

WO 2014/056713 (Unilever) discloses oral care compositions comprising calcium silicate hydrate comprising from 20 to 70% SiO₂ by weight of the calcium silicate hydrate, and physiologically acceptable carrier comprising humectants, surfactant, thickener or a mixture thereof.

None of the additional information above describes an oral care composition comprising a composite material wherein the composite material comprises a first component material comprising a biomineralization agent, and a second component material capable of reacting with phosphate ions to produce a calcium and phosphate *in situ* reaction product that adheres to tooth enamel, dentin or both and that is a reagent for hydroxyapatite formation. And especially such oral care composition has excellent tubule blockage efficacy and is more efficient for treating tooth hypersensitivity.

### Tests and Definitions

### Dentifrice

"Dentifrice" for the purposes of the present invention means a paste, powder, liquid, gum or other preparation for cleaning the teeth or other surfaces in the oral cavity.

### Tooth Paste

"Tooth paste" for the purpose of the present invention means a paste or gel dentifrice for use with a toothbrush. Especially preferred are tooth pastes suitable for cleaning teeth by brushing for about two minutes.

### Particle Size

"Particle size" for the purpose of the present invention means D50 particle size. The D50 particle size of a particulate material is the particle size diameter at which 50 wt% of the particles are larger in diameter and 50 wt% are smaller in diameter. Particle size can be measured, for example, by dynamic light scattering (DLS).

### pH

pH is quoted at atmospheric pressure and a temperature of 25°C. When referring to the pH of an oral care composition, this means the pH measured when 5 parts by weight of the composition is uniformly dispersed and/or dissolved in 20 parts by weight pure water at 25°C. In particular the pH may be measured by manually mixing 5 g oral care composition with 20 mL water for 30 s, then immediately testing the pH with indicator or a pH meter.

### Solubility

"Soluble" and "insoluble" for the purpose of the present invention means the solubility of a source (e.g., like calcium salts) in water at 25°C and atmospheric pressure. "Soluble" means a source that dissolves in water to give a solution with a concentration of at least 0.1 moles per litre. "Insoluble" means a source that dissolves in water to give a solution with a concentration of less than 0.001 moles per litre. "Slightly soluble", therefore, is defined to mean a source that dissolves in water to give a solution with a concentration of greater than 0.001 moles per litre and less than 0.1 moles per litre.

### Substantially Free

"Substantially free of" for the purpose of the present invention means less than 1.5%, and preferably less than 1.0%, and more preferably less than 0.75% and more preferably still less than 0.5%, and even more preferably less than 0.1% and most preferably from 0.0 to 0.01% by weight, based on total weight of the oral care composition, including all ranges subsumed therein.

### Dual-Phase

"Dual-Phase" for the purpose of the present invention means a composition having two independent phases which are physically separate.

### Anhydrous Composition

"Anhydrous composition" for the purpose of the present invention means the water content of the composition is less than 1.5%, preferably from 0.0 to 0.75% by total weight of the oral care composition.

### Viscosity

Viscosity of a tooth paste is the value taken at room temperature (25°C) with a Brookfield Viscometer, Spindle No.4 and at a speed of 5 rpm. Values are quoted in centipoises (cP=mPa.s) unless otherwise specified.

### Remineralization

"Remineralization" for the purpose of the present invention means *in situ* (i.e. in the oral cavity) generation of calcium phosphate on teeth (including layers on teeth from 10 nm to 20 microns, and preferably from 75 nm to 10 microns, and most preferably, from 150 nm to 5 microns thick including all ranges subsumed therein) to reduce the likelihood of tooth sensitivity, tooth decay, regenerate enamel and/or improve the appearance of teeth by whitening through the generation of such new calcium phosphate.

### Miscellaneous

Except in the examples, or where otherwise explicitly indicated, all numbers in this description indicating amounts of material or conditions of reaction, physical properties of materials and/or use may optionally be understood as modified by the word "about".

All amounts are by weight of the final oral care composition, unless otherwise specified. It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### Summary of the Invention

In a first aspect, the present invention is directed to an oral care composition comprising:
a) a composite material;
b) a physiologically acceptable carrier;
wherein the composite material comprises:
(i) a first component material comprising a biomineralization agent; and
(ii) a second component material capable of reacting with phosphate ions to produce a calcium and phosphate *in situ* reaction product that adheres to tooth enamel, dentin or both and that is a reagent for hydroxyapatite formation; and
wherein the biomineralization agent comprises amorphous calcium phosphate, α-tricalcium phosphate, β-tricalcium phosphate, calcium deficient hydroxyapatite (Ca₉(HPO₄)(PO₄)₅OH), dicalcium phosphate (CaHPO₄), dicalcium phosphate dehydrate (CaHPO₄·2H₂O), hydroxyapatite (Ca₁₀(PO₄)₆(OH)₂), octacalcium phosphate (Ca₈H₂(PO₄)₆·5H₂O), tetracalcium phosphate (Ca₄(PO₄)₂O) or a mixture thereof.

In a second aspect, the present invention is directed to a packaged oral care product comprising the oral care composition of the first aspect of this invention.

In a third aspect, the present invention is directed to a method of reducing sensitivity and/or remineralizing and/or whitening of teeth of an individual comprising the step of applying the oral care composition of any embodiment of the first aspect to at least one surface of the teeth of the individual.

In a forth aspect, the present invention is directed to a process for manufacturing an oral care composition comprising the steps of:
i) mixing the first component material with solvent to produce a slurry;
ii) heating the slurry to from 25°C to 95°C for 0.5 hours to 3 hours;
iii) adding reagent suitable for *in situ* generation of the second component material into the slurry to form a reaction mixture;
iv) recovering the composite material from the reaction mixture; and
v) combining the composite material with physiologically acceptable carrier.

The process of the forth aspect is particularly suitable for manufacturing the composition of the first aspect. Thus the present invention also provides the oral care composition of the first aspect obtained and/or obtainable by the process of the forth aspect of the invention.

All other aspects of the present invention will more readily become apparent upon considering the detailed description and examples which follow.

### Detailed Description

Now it has been found that an oral care composition comprising a composite material is more efficient for treating tooth hypersensitivity. The only limitation with respect to the composite material that may be used in this invention is that the same is suitable for use in the mouth. In addition, such composition can also enhance the tooth remineralization efficacy and/or the deposition of benefit agents on tooth surfaces to further benefit teeth of an individual.

The composite material is a compound comprising a first component material and a second component material, which are microscopically heterogeneous, but macroscopically homogeneous. The first component material is different from the second component material. Typically, the first component material of the composite material comprises a biomineralization agent that is suitable to improve the remineralization of teeth. Particularly suitable biomineralization agent comprises amorphous calcium phosphate, α-tricalcium phosphate, β-tricalcium phosphate, calcium deficient hydroxyapatite (Ca₉(HPO₄)(PO₄)₅OH), dicalcium phosphate (CaHPO₄), dicalcium phosphate dehydrate (CaHPO₄·2H₂O), hydroxyapatite (Ca₁₀(PO₄)₆(OH)₂), octacalcium phosphate (Ca₈H₂(PO₄)₆·5H₂O), tetracalcium phosphate (Ca₄(PO₄)₂O) or a mixture thereof.

Preferably, the first component material comprises hydroxyapatite. In an especially preferred embodiment, the first component material is at least 50% by weight of hydroxyapatite, and most preferably from 60 to 100% by weight of hydroxyapatite, based on total weight of the first component material. In another especially preferred embodiment, the first component material is hydroxyapatite.

Typically, the composite material comprises the first component material in an amount of from 3 to 98%, more preferably from 20 to 90%, and most preferably from 50 to 80% by weight of the composite material, based on total weight of the composite material and including all ranges subsumed therein.

The second component material comprises material suitable to adhere to tooth enamel, dentin or both. In a preferred embodiment, the second component material reacts with phosphate ions to produce a calcium and phosphate *in situ* reaction product that adheres to tooth enamel, dentin or both and that is a reagent for hydroxyapatite formation. "Adhere", as used herein, includes bonding and/or effectively coupling to tooth surfaces as a result of the interaction between the elements in the second component material and those in teeth.

Typically the second component material comprises the element calcium, and optionally, other metals like potassium, sodium, aluminium, magnesium as well as mixtures thereof whereby such optional metals are provided as, for example, sulfates, lactates, oxides, carbonates or silicates. Optionally, the second component material may be aluminium oxide or silica. In a preferred embodiment, the second component material is suitable to provide a biological or chemical improvement to teeth which is long term (e.g., results in hydroxyapatite formation). Preferably, the second component material employed comprises at least 50% by weight elemental calcium, and most preferably, at least 65% by weight elemental calcium based on total weight of metal in the second component material. In an especially preferred embodiment, the metal in the second component material is from 80 to 100% by weight of elemental calcium, based on total weight of metal in the second component material and including all ranges subsumed therein. In another especially preferred embodiment, the first component material and the second component material are slightly soluble or insoluble in water, but most preferably, insoluble in water.

In an especially desired embodiment, the second component material can comprise, for example, calcium oxide, calcium carbonate, calcium hydroxide, calcium sulfate, calcium carboxymethyl cellulose, calcium alginate, calcium salts of citric acid, calcium silicate, mixture of thereof or the like. In another desired embodiment, the calcium source in the second component material will comprise calcium silicate. These compounds are also suitable to include as reagents for calcium sources when making the composite material of this invention. Similar salts with sodium, magnesium, strontium, aluminium and potassium in lieu of calcium may, for example, be used as reagents to provide the anionic portion of the second component material during composite material manufacturing.

In yet, another preferred embodiment, the second component material can comprise the element calcium which originates from insoluble calcium silicate, present as the material calcium oxide-silica (CaO-SiO₂) as described in international patent applications published as WO 2008/015117 and WO 2008/068248.

When a calcium silicate material is employed as the second component material, the ratio of calcium to silicon (Ca:Si) may be from 1:10 to 3:1. The Ca:Si ratio is preferably from 1:5 to 3:1, and more preferably, from 1:3 to 3:1, and most preferably, from about 1:2 to 3:1. The calcium silicate may comprise mono-calcium silicate, bi-calcium silicate, or tri-calcium silicate whereby ratios of calcium to silicon (Ca:Si) should be understood to be atom ratios.

The preferred material employed in this invention to deliver calcium and generate second component material of the composite material may be in a crystalline or amorphous state. In an often preferred embodiment, the material to deliver calcium for the second component is in a mesoporous state, i.e. the source is a material having pores with diameters from 1 nm to 1 micron. Mesoporous calcium silicate (MCS) is often preferred.

The MCS which may be used in second component material in this invention can be made by combining a calcium salt (e.g., calcium chloride, calcium carbonate, calcium hydroxide), a silica reagent like silicate (e.g., sodium silicate, potassium silicate, tetraethyl orthosilicate or tetraethyl silicate) and a structure-directing agent to yield a solid suitable for calcinating. A more detailed description of the process that may be conducted to make the MCS suitable for use in this invention is described in the aforementioned international patent application published as WO 2008/015117.

In an often desired embodiment, the second component material may be formed from CaO-SiO₂.

The composite material is preferably particulate as this allows for maximum surface area for contact with dental tissue. Typically, the composite material has a size from 10 nm to less than 50 microns, and more preferably, from 50 nm to 10 microns, and most preferably from 100 nm to 5 microns, including all ranges subsumed therein.

Typically, the composite material comprises the first component material and the second component material in a weight ratio of from 1:10 to 10:1, more preferably from 1:3 to 5:1, and most preferably from 1:1 to 3:1.

Typically, the oral care composition of the present invention comprises from 0.25 to 60%, and more preferably, from 0.5 to 40%, and most preferably, from 1 to 30% by weight of the composite material, based on the total weight of the oral care composition and including all ranges subsumed therein.

In another especially preferred embodiment, the composite material may be added along with an additional metal source like a calcium source. Such an additional metal source may be identical to that which is used as the second component material of the composite material like calcium silicate, calcium oxide or a mixture thereof. When added, the additional metal source typically makes up from 0.1 to 50%, more preferably from 0.2 to 30%, most preferably from 1 to 20%, based on the total weight of the oral care composition and including all ranges subsumed therein.

In one embodiment, the oral care composition may be substantially free of phosphate source. This is especially preferred when the composition is a monophase hydrous composition (i.e. comprises greater than 1.5% water, preferably greater than 5% water, more preferably greater than 10% water and most preferably from 20 to 90% water by weight of the composition). Presence of composite material and phosphate sources in a monophase hydrous formulation can lead to premature reaction of the composite material and the phosphate and instability of the product.

For certain compositions, especially anhydrous compositions (i.e. compositions substantially free of water) or dual-phase hydrous compositions, it is preferable to compound a phosphate source in the oral composition to aid *in situ* generation of calcium phosphate.

The phosphate source that may be used in this invention is limited only to the extent that the same may be used in a composition suitable for use in the mouth. Illustrative examples of the types of phosphate source suitable for use in this invention include trisodium phosphate, monosodium dihydrogen phosphate, disodium hydrogen phosphate, tripotassium phosphate, monopotassium dihydrogen phosphate, dipotassium hydrogen phosphate, ammonium phosphate, diammonium hydrogen phosphate, ammonium dihydrogen phosphate, mixtures thereof or the like. The phosphate source is preferably one which is water soluble.

When used, the phosphate source typically makes up from 0.5 to 40%, and more preferably, from 1 to 30%, and most preferably, from 2 to 20% by weight of the oral care composition, based on total weight of the oral care composition and including all ranges subsumed therein. In a preferred embodiment, the phosphate source used is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios subsumed therein. In another preferred embodiment, the phosphate source used is or at least comprises monosodium dihydrogen phosphate.

The oral care composition preferably has a pH of greater than 5.0. If the pH of the composition is too low then it may lower the pH in the oral cavity such that generation of *in situ* calcium phosphate is retarded. Therefore, it is preferred that the pH of the oral care composition is in the range 5.5 to 11.0, more preferably 6.0 to 10.5 and most preferably 6.5 to 9.0.

The composition of the present invention is an oral care composition and typically comprises a physiologically acceptable carrier. The carrier preferably comprises at least surfactant, thickener, humectant or a combination thereof.

Preferably the oral care composition comprises a surfactant. Preferably the composition comprises at least 0.01% surfactant by weight of the composition, more preferably at least 0.1% and most preferably from 0.5 to 7%. Suitable surfactants include anionic surfactants, such as the sodium, magnesium, ammonium or ethanolamine salts of C₈ to C₁₈ alkyl sulfates (for example sodium lauryl sulfate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulfated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. More preferably the surfactant comprises or is anionic surfactant. The preferred anionic surfactants are sodium lauryl sulfate and/or sodium dodecylbenzene sulfonate. Most preferably the surfactant is sodium lauryl sulfate, sodium coco sulfate, cocamidopropyl betaine, sodium methyl cocoyl taurate or mixtures thereof.

Thickener may also be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum arabic, gum karaya, sodium alginate, carrageenan, guar, xanthan gum, Irish moss, starch, modified starch, silica based thickeners including silica aerogels, magnesium aluminum silicate (e.g., Veegum), Carbomers (cross-linked acrylates) and mixtures thereof.

Typically, xanthan gum and/or sodium carboxymethyl cellulose and/or a Carbomer is/are preferred. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

In another especially preferred embodiment, the thickener is xanthan gum.

Thickener typically makes up from 0.01 to about 10%, more preferably from 0.1 to 9%, and most preferably, from 0.1 to 5% by weight of the oral care composition, based on total weight of the composition and including all ranges subsumed therein.

When the oral care composition of this invention is a toothpaste or gel, the same typically has a viscosity from about 30,000 to 180,000 centipoise, and preferably, from 60,000 to 170,000 centipoise, and most preferably, from 65,000 to 165,000 centipoise.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of the oral care composition. More preferably, the carrier humectant makes up from 25 to 80%, and most preferably, from 30 to 60% by weight of the composition, based on total weight of the composition and including all ranges subsumed therein.

The oral care composition may further comprise benefit agents that are typically delivered to human teeth and/or the oral cavity including the gums to enhance or improve a characteristic of those dental tissues. The only limitation with respect to the benefit agents that may be used in this invention is that the same is suitable for use in the mouth. The benefit agents are present in the oral care composition in addition to the composite material that is included in the composition.

Typically the benefit agent is selected from optical agents, biomineralization agents, antibacterial agents, gum health agents, desensitizing agents, anti-calculus agents, freshness agents or mixtures thereof. Preferably, the benefit agent is selected from optical agents, antibacterial agents, gum health agents, freshness agents or mixtures thereof.

For example, optical agents such as coloring agents like whitening agents and pigments. Preferably, the pigment, when used, is violet or blue having a hue angle, h, in the CIELAB system of from 220 to 320 degrees. These pigments may be selected from one or more of those listed in the Colour Index International, listed as pigment blue 1 through to pigment blue 83, and pigment violet 1 through to pigment violet 56. In another preferred embodiment, the optical agents may be selected from one or more of mica, interference mica, boron nitride, poly(methyl methacrylate) flake, composite microspheres, titanium dioxide coated glass flake, inverse opal, cholesteric liquid crystal, photonic sphere, hollow sphere and zinc oxide. Antibacterial agents may be selected from one or more of metal salts where the metal is selected from zinc, copper, silver or a mixture thereof, triclosan, triclosan monophosphate, triclocarban, curcumin, quaternary ammonium compounds, bisbiguanides and long chain tertiary amines, preferably zinc salts including zinc oxide, zinc chloride, zinc acetate, zinc ascorbate, zinc sulphate, zinc nitrate, zinc citrate, zinc lactate, zinc peroxide, zinc fluoride, zinc ammonium sulfate, zinc bromide, zinc iodide, zinc gluconate, zinc tartarate, zinc succinate, zinc formate, zinc phenol sulfonate, zinc salicylate, zinc glycerophosphate or a mixture thereof. Gum health agents may be selected from one or more of anti-inflammatory agents, plaque buffers, biomolecules, proteinaceous materials, vitamin, plant extracts and curcumin. Freshness agents may be flavors selected from one or more of peppermint, spearmint, menthol, flora oil, clove oil and citrus oil.

The benefit agent is preferably particulate as this allows for maximum surface area for contact with dental tissue.

In a preferred embodiment, the benefit agent is a particulate whitening agent for tooth whitening.

Typically, the particulate whitening agent comprises a material suitable to physically and immediately improve characteristics of teeth and especially whiten teeth. In order to provide excellent whitening effect, the material is preferred to have a high refractive index of at least 1.9, more preferably at least 2.0, even more preferably at least 2.2, even more preferably still at least 2.4 and most preferably at least 2.5. The maximum refractive index of the material is not particularly limited but preferably up to 4.0. Preferably, the material has a refractive index ranging from 1.9 to 4.0.

Particularly suitable materials are metal compounds and preferred are compounds where the metal is selected from zinc (Zn), titanium (Ti), zirconium (Zr) or a combination thereof. Preferably, the metal compound is (or at least comprises) a metal oxide such as titanium dioxide (TiO₂), zinc oxide (ZnO), zirconium dioxide (ZrO₂) or a combination thereof. In addition, the particulate whitening agent can also comprise non-metal oxides such as strontium titanate and zinc sulfide.

In a preferred embodiment, the particulate whitening agent comprises metal oxides, non-metal oxides or a combination thereof in an amount of at least 50% by weight of the whitening agent and more preferably at least 70%, more preferably still from 80 to 100% and most preferably from 85 to 95%. In an especially preferred embodiment, the particulate whitening agent is at least 50% by weight titanium dioxide, and most preferably, from 60 to 100% by weight titanium dioxide, based on total weight of the whitening agent and including all ranges subsumed therein. In another especially preferred embodiment, the particulate whitening agents are slightly soluble or insoluble in water, but most preferably, insoluble in water.

In a preferred embodiment, the particulate whitening agents are composite particles. "Composite particles" as used herein, means a particle comprising a first component core and a second component coating wherein the core and coating are composed of different materials. The refractive index of a composite particle comprising more than one material can be calculated based on the refractive indices and volume fractions of the constituents using effective medium theory, as is described for example in WO 2009/023353.

The composite particle comprises a first component core and a second component coating. Typically, the core of the composite particle comprises a material suitable to physically and immediately improve characteristics of teeth and especially whiten teeth. In order to provide excellent whitening effect, the material is preferred to have a high refractive index of at least 1.9, more preferably at least 2.0, even more preferably at least 2.2, even more preferably still at least 2.4 and most preferably at least 2.5. The maximum refractive index of the material is not particularly limited but preferably up to 4.0. Preferably, the material has a refractive index ranging from 1.9 to 4.0.

Particular suitable materials are metal compounds and preferred are compounds where the metal is selected from zinc (Zn), titanium (Ti), zirconium (Zr) or a combination thereof. Preferably, the metal compound is (or at least comprises) a metal oxide such as titanium dioxide (TiO₂), zinc oxide (ZnO), zirconium dioxide (ZrO₂) or a combination thereof. In addition, the core of the composite particle can also comprise non-metal oxides such as strontium titanate and zinc sulfide. In an especially preferred embodiment, the core is at least 50% by weight titanium dioxide, and most preferably, from 60 to 100% by weight titanium dioxide, based on total weight of the first component core.

Typically the coating material comprises the element calcium, and optionally, other metals like potassium, sodium, aluminium, magnesium as well as mixtures thereof whereby such optional metals are provided as, for example, sulphates, lactates, oxides, carbonates or silicates. In an especially desired embodiment, the second component coating can comprise, for example, calcium phosphate, calcium oxide, calcium carbonate, calcium hydroxide, calcium sulphate, calcium carboxymethyl cellulose, calcium alginate, calcium salts of citric acid, calcium silicate, mixture thereof or the like. In another desired embodiment, the calcium source in the coating comprises calcium silicate.

Usually, at least 30% of the outer surface area of the first component core is coated with the second component coating, preferably at least 50% of the core is coated with the coating, most preferably, from 70 to 100% of the outer surface area of the first component core is coated with the second component coating.

In an especially preferred embodiment, the particulate whitening agent is titanium dioxide coated with calcium silicate.

The particulate whitening agent according to the present invention can be of different sizes and shapes. The particles may be of spherical, platelet or irregular shape form. The diameter of the particulate whitening agent is often from 10 nm to less than 50 microns, and preferably, from 75 nm to less than 10 microns. In an especially preferred embodiment, the diameter of particles is from 100 nm to 5 microns, including all ranges subsumed therein. Particle size can be measured, for example, by dynamic light scattering (DLS). For composite particles, in a preferred embodiment, at least 40%, and preferably, at least 60%, and most preferably, from 75 to 99.5% of the diameter of the composite particle is the core, including all ranges subsumed therein.

The oral care composition of the present invention may comprise a single benefit agent or a mixture of two or more benefit agents. Typically, the benefit agent is present in an amount from 0.25 to 60%, and more preferably, from 0.5 to 40%, and most preferably, from 1 to 30% by total weight of the oral care composition and including all ranges subsumed therein.

The oral care composition of the present invention may contain a variety of other ingredients which are common in the art to enhance physical properties and performance. These ingredients include preservatives, pH-adjusting agents, sweetening agents, particulate abrasive materials, polymeric compounds, buffers and salts to buffer the pH and ionic strength of the compositions, and mixtures thereof. Such ingredients typically and collectively make up less than 20% by weight of the composition, and preferably, from 0.0 to 15% by weight, and most preferably, from 0.01 to 12% by weight of the composition, including all ranges subsumed therein.

The oral care composition of this invention can be used in a method of benefiting teeth of an individual comprising applying in the composition to at least one surface of the teeth of an individual, said benefits includes reduced sensitivity, remineralization, whitening and combinations thereof. The oral care composition of this invention may additionally or alternatively be for use as a medicament and/or used in the manufacture of a medicament for providing an oral care benefit as described herein, such as for reducing sensitivity of the teeth of an individual. Alternatively and preferably, the use is non-therapeutic.

In the event an oral care product with a phosphate source is desired, in one preferred embodiment, the oral care composition is a monophase anhydrous composition that is substantially free of water to prevent the premature reaction between the composite material and the phosphate source.

In another preferred embodiment, the oral care composition is a dual-phase composition comprising a first phase and a second phase, wherein the composite material is present in the first phase and the phosphate source is present in the second phase. The delivery of the two independent phases to the teeth may be simultaneous or sequential. In a preferred embodiment, the phases are delivered simultaneously. When a dual-phase oral care composition is desired, water may act as a carrier (along with thickeners and/or additional carriers herein described) and make up the balance of each phase in the dual-phase composition.

When a dual-phase composition is used, the first phase and the second phase should not come into contact with each other until dispensed for use. In use, it is preferably to combine the two phases to form a mix prior to their application to the teeth. Typically, the weight ratio of the first phase and the second phase in this mix is from 1:3 to 10:1, more preferably from 1:2 to 7:1, most preferably from 1:1.5 to 5:1.

Typically, the dual-phase composition is delivered by a dual-tube having a first compartment for the first phase and a second compartment for the second phase, which allows for co-extrusion of the two phases.

In a preferred embodiment, such a dual-tube has one of the compartments surrounding the other. In such embodiments, one phase is present as a sheath, surrounding the other phase in the core. In an especially preferred embodiment, the core is the first phase and the sheath is the second phase.

In another preferred embodiment, such a dual-tube has the two compartments side by side within the same tube. In such embodiments, the two phases are extruded from the tube as one, such extrusion being termed "contact extrusion". A pump head may be used in such a dual-tube for squeezing the two phases from the tube as one.

The dual-phase oral care composition may be a gel composition that comprises two independent gel phases, one is the first phase and the other is the second phase. The means of delivery may involve a cotton rod, or a tray, onto which the first phase and the second phase are applied, prior to the tray being placed in contact with the teeth.

The composite material of this invention may be prepared by mixing the first component material with solvent to produce a slurry. The suitable solvent comprises water, low molecular weight alcohols like C₂₋₆ alkanols, low molecular weight ketones like acetone or mixtures thereof. Preferably, the solvent is water. The slurry should be heated from 25 to 95°C, and preferably from 40 to 90°C, and most preferably from 50 to 80°C, including all ranges subsumed therein. While heating, reagent for anionic portion of the second component material should be added to the heated slurry. After addition of the anionic portion reagent, the slurry should be incubated from 0.5 hours to 3 hours, and preferably from 1 hour to 2.5 hours at an alkaline pH (preferably from pH 7 to pH 11, and most preferably from pH 8 to pH 10). Suitable reagent for anionic portion of the second component material comprises sodium silicate, potassium silicate, sodium alginate, sodium sulfate, tetraethyl orthosilicate or mixtures thereof. Reagent to supply cationic portion of the second component material, like calcium ions, should then be added to the slurry. Suitable reagent for cationic portion of the second component material comprises calcium gluconate, calcium oxide, calcium lactate, calcium acetate, calcium hydroxide, calcium sulfate, calcium carboxymethyl cellulose, calcium chloride, potassium chloride, magnesium chloride, strontium chloride, aluminum chloride or mixtures thereof. The resulting mixture should be incubated from 0.5 hours to 3 hours, preferably from 1 hour to 2.5 hours and aged overnight. The resulting composite material may be recovered by conventional techniques including filtration and/or centrifugation and subsequently dried.

The oral care compositions of this invention are prepared by conventional methods of making oral care compositions, which include mixing the ingredients under moderate shear and atmospheric pressure.

Typically the composition will be packaged. In tooth paste or gel form, the composition may be packaged in a conventional plastic laminate, metal tube or a single compartment dispenser. The same may be applied to dental surfaces by any physical means, such as a toothbrush, fingertip or by an applicator directly to the sensitive area.

The composition can be effective even when used in an individual's daily oral hygiene routine. For example, the composition may be brushed onto the teeth. The composition may, for example, be contacted with the teeth for a time period of one second to 20 hours. More preferably from 1 s to 10 hours, more preferably still from 10 s to 1 hour and most preferably from 30 s to 5 minutes. The composition may be used daily, for example for use by an individual once, twice or three times per day. When the oral care composition is a dual-phase composition, the two phases of the composition are mixed during application. The mixed phases are typically left on the teeth for from 3 minutes to 10 hours, more preferably from 3 minutes to 8 hours. The application may be carried out daily.

The following examples are provided to facilitate an understanding of the present invention. The examples are not provided to limit the scope of the claims.

### Examples

### Example 1

This example demonstrates the improved blockage of dentinal tubules by using a composite material. All ingredients are expressed by weight percent of the total formulation, and as level of active ingredient.

**TABLE 1**

| Ingredient | Samples | | | |
|---|---|---|---|---|
| | **1** | **2** | **3** | **4** |
| HAP | 5.0 | -- | 3.6 | -- |
| Calcium silicate | -- | -- | 1.4 | 5.0 |
| HAP/ calcium silicate composite material | -- | 5.0 | -- | -- |
| Glycerin | 95.0 | 95.0 | 95.0 | 95.0 |

### Methods

### Preparation of a composite material

5 g of hydroxyapatite powder was added in 100 mL distilled water to form a slurry. After homogenization at 5000 rpm for 10 mins, the slurry was continuously stirred whilst maintained at a temperature of 70°C via IKA stirring machine. After 0.5 hours, 16.7 mL 1M sodium silicate was added dropwise in the slurry and the pH of the slurry was maintained in a range from 8.5-9.5 using 1M hydrochloric acid. After the addition of sodium silicate, the slurry was incubated for 2 hours. Then the heating was turned off and the slurry was kept stirring. 1.24g calcium hydroxide powder was added into the slurry then the resulted mixture was stirred for 2 hours and aged overnight. The composite material was washed and collected by centrifugation method.

### Samples Preparation

To evaluate blockage efficacy of dentinal tubules, the test sample was mixed with water at a ratio of 4g to 8mL water to make a slurry. Human dentine discs were eroded by 37% phosphoric acid for 1 min, then they were treated with different slurries via brushing following the same protocol. Eight human dentine discs were separated into four groups (n=2). The dentine discs were brushed with the slurry under a tooth brushing machine equipped with toothbrushes. The load of the tooth brushing was 170 g +/-5 g and the automatic brushing operated at a speed of 150 rpm. After brushing for 1 min, the dentine discs were soaked in toothpaste slurry for 1 min. Then the dentine discs were rinsed with de-ionized water. Then the dentine discs were rinsed with distilled water and placed in simulated oral fluid (SOF) under the condition of a shaking water bath at 37°C and 60.0 rpm for at least 3 hours. The brushing was repeated three times for one day, then the dentine discs were kept in SOF overnight(>12 hours) in a shaking water bath at 37°C to mimic oral environment. The dentine samples were brushed for 3 and 7 times.

Simulated oral fluid was made by combining the ingredients in Table 2:

**TABLE 2**

| Ingredient | Amount/g |
|---|---|
| NaCl | 16.07 |
| NaHCOs | 0.7 |
| KCI | 0.448 |
| K₂HPO₄*3H₂O | 3.27 |
| MgCl₂*6H₂O | 0.0622 |
| 1M HCI | 40 mL |
| CaCI2 | 0.1998 |
| Na₂SO₄ | 0.1434 |
| Buffer | Adjust pH to 7.0 |
| Water | Balance to 2 L |

### Scoring Standard for Tubules Blockage

Regardless of the original shape of the dentine discs, a square (with a size of 4mm x 4mm) is selected and one image is captured under 50x magnification. Within this square, five spots (each with a size of 150 µm x 150 µm, one in the middle, and one in every corner) are selected and observed under 1000x magnification. The blockage of tubules are accessed following the standards described in Table 3. The measurement is carried out for the two dentine discs of each test group.

**TABLE 3**

| **Score** | **Tubules Blockage** |
|---|---|
| 0 | All dentinal tubules are open. |
| 1 | <20% of dentinal tubules are fully blocked. |
| 2 | 20 to 50% of dentinal tubules are fully blocked. |
| 3 | 50 to 80% of dentinal tubules are fully blocked. |
| 4 | 80-100% of dentinal tubules are fully blocked. |
| 5 | All dentinal tubules are fully blocked. |

### Results

SEM (Scanning Electron Microscopy) images of the dentine discs were taken after 3 and 7 times of brushings. The images were analyzed and scored. The results are summarized in Table 4 (error represents standard deviation for duplicate measurements).

**TABLE 4**

| Tubules Blockage Score | Samples | | | |
|---|---|---|---|---|
| | **1** | **2** | **3** | **4** |
| 3 brushings | 2.0 ± 0.82 | 4.7 ± 0.48 | 2.4 ± 0.52 | 1.1 ± 0.32 |
| 7 brushings | 3.9 ± 0.74 | 4.8 ± 0.42 | 3.6 ± 0.52 | 2.0 ± 0.67 |

The results showed that Sample 2 comprising composite material had significantly better (p<0.01) tubule blockage efficacy compared to other samples after 3 or 7 times of brushings. SEM images clearly showed that almost all the dentinal tubules were extensively blocked after treated with Sample 2.

### Example 2

This example demonstrates the improved blockage of dentinal tubules by using a composite material in formulations. All ingredients are expressed by weight percent of the total formulation, and as level of active ingredient.

**TABLE 5**

| Ingredients | Samples | |
|---|---|---|
| | 5 | 6 |
| Glycerin | 66.84 | 66.84 |
| Sodium monofluorophosphate | 1.11 | 1.11 |
| Sodium saccharin | 0.25 | 0.25 |
| Calcium silicate^{a} | 15.00 | -- |
| HAP/calcium silicate composite material | -- | 15.00 |
| Monosodium dihydrogen phosphate | 3.20 | 3.20 |
| Trisodium phosphate | 3.80 | 3.80 |
| Sodium lauryl sulfate | 2.00 | 2.00 |
| Silica abrasive^{b} | 6.00 | 6.00 |
| Silica abrasive^{c} | 0.50 | 0.50 |
| Xanthan gum | 0.10 | 0.10 |
| Flavour | 1.20 | 1.20 |

| | | |
|---|---|---|
| a. Commercially available calcium silicate (CaSiO₃) under the trade name Sorbosil CA40 from PQ Corporation. b. Commercially available silica under the trade name Sorbosil AC77 from PQ Corporation. c. Commercially available silica under the trade name Sorbosil AC43 from PQ Corporation. | | |

Comparative sample A is a commercial anti-sensitivity toothpaste claimed to have good tooth repair credentials, which comprises glycerin, PEG-400, silica, calcium sodium phosphosilicate , sodium lauryl sulphate, sodium monofluorophosphate, aroma, titanium dioxide, carbomer, potassium acesulfame.

### Methods

The same protocol was used to evaluate the blockage efficacy of dentinal tubules as described in Example 1. The dentine discs were brushed for 3 and 7 times.

### Results

SEM (Scanning Electron Microscopy) images of the dentine discs were taken after 3 and 7 times of brushings. The images were analyzed and scored. The results are summarized in Table 6 (error represents standard deviation for duplicate measurements).

**TABLE 6**

| Tubules Blockage Score | Samples | | |
|---|---|---|---|
| | **A** | **5** | **6** |
| 3 brushings | 1.5 ± 0.71 | 1.3 ± 0.48 | 2.3 ± 0.95 |
| 7 brushings | 2.2 ± 1.23 | 2.2 ± 0.79 | 3.9 ± 0.57 |

After 7 brushings, SEM images of the dentine disks were taken. Sample 6 comprising composite material had significantly better (p<0.01) tubule blockage efficacy than other samples. From the top view of the SEM images, it can be seen that Sample 6 showed good deposition on dentine surfaces and the dentinal tubules were extensively blocked.

### Example 3

This example demonstrates the improved deposition on tooth surfaces by using a composite material in formulations. Samples used here were Samples A, 5 and 6 as described in Example 2.

### Methods

The same protocol was used to evaluate the blockage efficacy of dentinal tubules as described in Example 1 except that bovine enamel blocks were used instead of dentine discs. The enamel blocks were brushed for 28 times.

### Results

SEM images of the enamel block surfaces were taken after 28 brushings. From the top view of the SEM images, it can be seen that Sample 6 comprising the composite material showed better deposition on enamel surfaces than other samples. The corresponding cross-section SEM images further showed that a much thicker new layer was formed on the enamel surfaces after treatment with Sample 6 compared to that treated with Sample 5, while the enamel blocks treated with Sample A did not have a layer formed on their surfaces. Analysis using EDX identified the elements of Si, Ca and P within the new layer, indicating the composite material induced good tooth remineralization.

## Claims

1. An oral care composition comprising:
a) a composite material;
b) a physiologically acceptable carrier;
wherein the composite material comprises:
(i) a first component material comprising a biomineralization agent; and
(ii) a second component material capable of reacting with phosphate ions to produce a calcium and phosphate *in situ* reaction product that adheres to tooth enamel, dentin or both and that is a reagent for hydroxyapatite formation;
wherein the biomineralization agent comprises amorphous calcium phosphate, α-tricalcium phosphate, β-tricalcium phosphate, calcium deficient hydroxyapatite (Ca₉(HPO₄)(PO₄)₅OH), dicalcium phosphate (CaHPO₄), dicalcium phosphate dehydrate (CaHPO₄·2H₂O), hydroxyapatite (Ca₁₀(PO₄)₆(OH)₂), octacalcium phosphate (Ca₈H₂(PO₄)₅·5H₂O), tetracalcium phosphate (Ca₄(PO₄)₂O) or a mixture thereof; and
wherein the second component material comprises calcium oxide, calcium carbonate, calcium hydroxide, calcium sulfate, calcium carboxymethyl cellulose, calcium alginate, calcium salts of citric acid, calcium silicate or a mixture thereof.

2. The oral care composition according to claim 1, wherein the biomineralization agent is hydroxyapatite.

3. The oral care composition according to claim 1 or claim 2, wherein the composite material comprises the first component material in an amount of from 3 to 98%, preferably from 20 to 90% by weight of the composite material.

4. The oral care composition according to any of the preceding claims, wherein the second component material comprises calcium silicate.

5. The oral care composition according to any of the preceding claims, wherein the composite material has a particle size in the range from 50 nm to 10 microns, preferably from 100 nm to 5 microns.

6. The oral care composition according to any of the preceding claims, wherein the composite material comprises the first component material and the second component material in a weight ratio of from 1:10 to 10:1, preferably from 1:3 to 5:1.

7. The oral care composition according to any of the preceding claims, wherein the composite material is present in an amount from 0.25 to 60% by weight of composition, preferably from 0.5 to 40% by weight of the composition.

8. The oral care composition according to any of the preceding claims, wherein the composition further comprises phosphate source selected from trisodium phosphate, monosodium dihydrogen phosphate, disodium hydrogen phosphate, ammonium phosphate, diammonium hydrogen phosphate, ammonium dihydrogen phosphate, tripotassium phosphate, monopotassium dihydrogen phosphate, dipotassium hydrogen phosphate or a mixture thereof, preferably trisodium phosphate, monosodium dihydrogen phosphate or mixtures thereof.

9. The oral care composition according to any of the preceding claims, wherein the composition further comprises a metal source, preferably calcium silicate, calcium oxide or a mixture thereof.

10. The oral care composition according to any of the preceding claims, wherein the composition further comprises a benefit agent, preferably titanium dioxide coated with calcium silicate.

11. The oral care composition according to any of the preceding claims, wherein the composition is a monophase anhydrous composition.

12. The oral care composition according to any one of the claims 8 to 10, wherein the oral care composition is a dual-phase composition comprising a first phase and a second phase, wherein the composite material is present in the first phase and the phosphate source is present in the second phase.

13. A process for manufacturing an oral care composition according to any of the preceding claims comprising the steps of:
i) mixing the first component material with solvent to produce a slurry;
ii) heating the slurry to from 25°C to 95°C for 0.5 hours to 3 hours;
iii) adding reagent suitable for *in situ* generation of the second component material into the slurry to form a reaction mixture;
iv) recovering the composite material from the reaction mixture; and
v) combining the composite material with physiologically acceptable carrier.

14. The process according to claim 13, wherein the reagent suitable for anionic portion of the second component material comprises sodium silicate, potassium silicate, sodium alginate, sodium sulfate, tetraethyl orthosilicate or mixtures thereof and wherein the reagent suitable for cationic portion of the second component material comprises calcium gluconate, calcium oxide, calcium lactate, calcium acetate, calcium hydroxide, calcium sulfate, calcium carboxymethyl cellulose, calcium chloride, potassium chloride, magnesium chloride, strontium chloride, aluminum chloride or mixtures thereof.

15. A method for whitening of teeth of an individual comprising applying the composition as claimed in any one of claims 1 to 12 to at least one surface of the teeth of the individual.

16. An oral care composition according to any one of the preceding claims 1 to 12 or an oral care composition obtainable by a process according to claim 13 or 14 for use as a medicament.

17. An oral care composition according to any one of the preceding claims 1 to 12 or obtainable by a process according to any one of the claims 13 or 14 for use in reducing tooth sensitivity.

18. An oral care composition according to any one of the preceding claims 1 to 12 or obtainable by a process according to claim 13 or 14 for use in remineralizing of teeth of an individual comprising the step of applying the composition to at least one of the teeth of the individual.

## Patentansprüche

1. Mundpflegezusammensetzung, umfassend:
a) ein Kompositmaterial;
b) einen physiologisch verträglichen Träger;
wobei das Kompositmaterial umfasst:
(i) ein Material einer ersten Komponente, umfassend ein Biomineralisierungsmittel; und
(ii) ein Material einer zweiten Komponente, das mit Phosphationen reagieren kann, um ein in situ Calcium- und Phosphat-Reaktionsprodukt zu erzeugen, das an Zahnschmelz, Dentin oder beiden haftet und ein Reagenz für Hydroxyapatitbildung ist;
wobei das Biomineralisierungsmittel amorphes Calciumphosphat, α-Tricalciumphosphat, β-Tricalciumphosphat, calciumarmes Hydroxyapatit (Ca₉(HPO₄)(PO₄)₅OH), Dicalciumphosphat (CaHPO₄), Dicalciumphosphatdihydrat (CaHPO₄·2H₂O), Hydroxyapatit (Ca₁₀(PO₄)₆(OH)₂), Octacalciumphosphat (Ca₈H₂ (PO₄)₆·5H₂O), Tetracalciumphosphat (Ca₄(PO₄)₂O) oder eine Mischung davon umfasst; und wobei das Material der zweiten Komponente Calciumoxid, Calciumcarbonat, Calciumhydroxid, Calciumsulfat, Calciumcarboxymethylcellulose, Calciumalginat, Calciumsalze der Citronensäure, Calciumsilicat oder eine Mischung davon umfasst.

2. Mundpflegezusammensetzung nach Anspruch 1, wobei das Biomineralisierungsmittel Hydroxyapatit ist.

3. Mundpflegezusammensetzung nach Anspruch 1 oder Anspruch 2, wobei das Kompositmaterial das Material der ersten Komponente in einer Menge von 3 bis 98 Gewichts-%, bevorzugt 20 bis 90 Gewichts-%, des Kompositmaterials umfasst.

4. Mundpflegezusammensetzung nach irgendeinem der vorhergehenden Ansprüche, wobei das Material der zweiten Komponente Calciumsilicat umfasst.

5. Mundpflegezusammensetzung nach irgendeinem der vorhergehenden Ansprüche, wobei das Kompositmaterial eine Teilchengröße im Bereich von 50 nm bis 10 Mikron, bevorzugt von 100 nm bis 5 Mikron, aufweist.

6. Mundpflegezusammensetzung nach irgendeinem der vorhergehenden Ansprüche, wobei das Kompositmaterial das Material der ersten Komponente und das Material der zweiten Komponente in einem Gewichtsverhältnis von 1:10 bis 10:1, bevorzugt von 1:3 bis 5:1, umfasst.

7. Mundpflegezusammensetzung nach irgendeinem der vorhergehenden Ansprüche, wobei das Kompositmaterial in einer Menge von 0,25 bis 60 Gewichts-% der Zusammensetzung, bevorzugt von 0,5 bis 40 Gewichts-% der Zusammensetzung, vorliegt.

8. Mundpflegezusammensetzung nach irgendeinem der vorhergehenden Ansprüche, wobei die Zusammensetzung ferner eine Phosphatquelle umfasst, die ausgewählt ist aus Trinatriumphosphat, Mononatriumdihydrogenphosphat, Dinatriumhydrogenphosphat, Ammoniumphosphat, Diammoniumhydrogenphosphat, Ammoniumdihydrogenphosphat, Trikaliumphosphat, Monokaliumdihydrogenphosphat, Dikaliumhydrogenphosphat oder eine Mischung davon, bevorzugt Trinatriumphosphat, Mononatriumdihydrogenphosphat oder Mischungen davon.

9. Mundpflegezusammensetzung nach irgendeinem der vorhergehenden Ansprüche, wobei die Zusammensetzung ferner eine Metallquelle, bevorzugt Calciumsilicat, Calciumoxid oder eine Mischung davon, umfasst.

10. Mundpflegezusammensetzung nach irgendeinem der vorhergehenden Ansprüche, wobei die Zusammensetzung ferner ein Vorteil verleihendes Mittel umfasst, bevorzugt Titandioxid, das mit Calciumsilicat beschichtet ist.

11. Mundpflegezusammensetzung nach irgendeinem der vorhergehenden Ansprüche, wobei die Zusammensetzung eine einphasige wasserfreie Zusammensetzung ist.

12. Mundpflegezusammensetzung nach irgendeinem der Ansprüche 8 bis 10, wobei die Mundpflegezusammensetzung eine Zweiphasenzusammensetzung ist, die eine erste Phase und eine zweite Phase umfasst, wobei das Kompositmaterial in der ersten Phase vorhanden ist und die Phosphatquelle in der zweiten Phase vorhanden ist.

13. Verfahren zur Herstellung einer Mundpflegezusammensetzung nach irgendeinem der vorhergehenden Ansprüche, umfassend die Schritte:
i) Mischen des Materials der ersten Komponente mit Lösungsmittel, um eine Aufschlämmung herzustellen;
ii) Erhitzen der Aufschlämmung auf 25 °C bis 95 °C für 0,5 Stunden bis 3 Stunden;
iii) Hinzufügen eines Reagenzes, das zur in situ Erzeugung des Materials der zweiten Komponente geeignet ist, in die Aufschlämmung, um ein Reaktionsgemisch zu bilden;
iv) Gewinnen des Kompositmaterials aus dem Reaktionsgemisch; und
v) Kombinieren des Kompositmaterials mit physiologisch verträglichem Träger.

14. Verfahren nach Anspruch 13, wobei das Reagenz, das für den anionischen Teil des Materials der zweiten Komponente geeignet ist, Natriumsilicat, Kaliumsilicat, Natriumalginat, Natriumsulfat, Tetraethylorthosilicat oder Mischungen davon umfasst, und wobei das Reagenz, das für den kationischen Teil des Materials der zweiten Komponente geeignet ist, Calciumgluconat, Calciumoxid, Calciumlactat, Calciumacetat, Calciumhydroxid, Calciumsulfat, Calciumcarboxymethylcellulose, Calciumchlorid, Kaliumchlorid, Magnesiumchlorid, Strontiumchlorid, Aluminiumchlorid oder Mischungen davon umfasst.

15. Verfahren zur Aufhellung von Zähnen eines Individuums, umfassend das Aufbringen der Zusammensetzung, wie in irgendeinem der Ansprüche 1 bis 12 beansprucht, auf mindestens eine Oberfläche der Zähne des Individuums.

16. Mundpflegezusammensetzung nach irgendeinem der vorhergehenden Ansprüche 1 bis 12 oder eine Mundpflegezusammensetzung, erhältlich nach einem Verfahren nach Anspruch 13 oder Anspruch 14, zur Verwendung als ein Arzneimittel.

17. Mundpflegezusammensetzung nach irgendeinem der vorhergehenden Ansprüche 1 bis 12 oder erhältlich durch ein Verfahren nach irgendeinem der Ansprüche 13 oder 14 zur Verwendung bei der Verringerung von Zahnempfindlichkeit.

18. Mundpflegezusammensetzung nach irgendeinem der vorhergehenden Ansprüche 1 bis 12 oder erhältlich nach einem Verfahren nach Anspruch 13 oder 14 zur Verwendung bei der Remineralisierung von Zähnen eines Individuums, umfassend den Schritt des Aufbringens der Zusammensetzung auf mindestens einen der Zähne des Individuums.

## Revendications

1. Composition pour le soin oral comprenant :
a) un matériau composite ;
b) un support physiologiquement acceptable ;
dans laquelle le matériau composite comprend :
i) un matériau de premier constituant comprenant un agent de biominéralisation ; et
ii) un matériau de second constituant pouvant réagir avec des ions phosphate pour produire un produit de réaction *in situ* de calcium et phosphate qui adhère à l'émail de la dent, la dentine ou les deux et qui est un réactif pour la formation d'hydroxyapatite ;
dans laquelle l'agent de biominéralisation comprend du phosphate de calcium amorphe, phosphate d'a-tricalcium, phosphate de β-tricalcium, hydroxyapatite pauvre en calcium (Ca₉(HPO₄)(PO₄)₅OH), phosphate de dicalcium (CaHPO₄), phosphate de dicalcium dihydraté (CaHPO₄.2H₂O), hydroxyapatite (Ca₁₀(PO₄)₆(CH)₂), phosphate d'octacalcium (Ca₈H₂(PO₄)₆.5H₂O), phosphate de tétracalcium (Ca₄(PO₄)₂O), ou un mélange de ceux-ci ; et
dans laquelle le matériau de second constituant comprend de l'oxyde de calcium, carbonate de calcium, hydroxyde de calcium, sulfate de calcium, calcium carboxyméthylcellulose, alginate de calcium, sels de calcium d'acide citrique, silicate de calcium ou un mélange de ceux-ci.

2. Composition pour le soin oral selon la revendication 1, dans laquelle l'agent de biominéralisation est l'hydroxyapatite.

3. Composition pour le soin oral selon la revendication 1 ou revendication 2, dans laquelle le matériau composite comprend le matériau de premier constituant dans une quantité de 3 à 98 %, de préférence de 20 à 90 % en masse du matériau composite.

4. Composition pour le soin oral selon l'une quelconque des revendications précédentes, dans laquelle le matériau de second constituant comprend du silicate de calcium.

5. Composition pour le soin oral selon l'une quelconque des revendications précédentes, dans laquelle le matériau composite présente une taille de particule dans l'intervalle de 50 nm à 10 microns, de préférence de 100 nm à 5 microns.

6. Composition pour le soin oral selon l'une quelconque des revendications précédentes, dans laquelle le matériau composite comprend le matériau de premier constituant et le matériau de second constituant dans un rapport massique de 1:10 à 10:1, de préférence de 1:3 à 5:1.

7. Composition pour le soin oral selon l'une quelconque des revendications précédentes, dans laquelle le matériau composite est présent dans une quantité de 0,25 à 60 % en masse de la composition, de préférence de 0,5 à 40 % en masse de la composition.

8. Composition pour le soin oral selon l'une quelconque des revendications précédentes, dans laquelle la composition comprend de plus une source de phosphate choisie parmi le phosphate de trisodium, dihydrogénophosphate de monosodium, hydrogénophosphate de disodium, phosphate d'ammonium, hydrogénophosphate de diammonium, dihydrogénophosphate d'ammonium, phosphate de tripotassium, dihydrogénophosphate de monopotassium, hydrogénophosphate de dipotassium ou un mélange de ceux-ci, de préférence phosphate de trisodium, dihydrogénophosphate de monosodium ou des mélanges de ceux-ci.

9. Composition pour le soin oral selon l'une quelconque des revendications précédentes, dans laquelle la composition comprend de plus une source de métal, de préférence du silicate de calcium, oxyde de calcium ou un mélange de ceux-ci.

10. Composition pour le soin oral selon l'une quelconque des revendications précédentes, dans laquelle la composition comprend de plus un agent bénéfique, de préférence du dioxyde de titane revêtu avec du silicate de calcium.

11. Composition pour le soin oral selon l'une quelconque des revendications précédentes, dans laquelle la composition est une composition anhydre monophase.

12. Composition pour le soin oral selon l'une quelconque des revendications 8 à 10, dans laquelle la composition pour le soin oral est une composition à phase double comprenant une première phase et une seconde phase, dans laquelle le matériau composite est présent dans la première phase et la source de phosphate est présente dans la seconde phase.

13. Procédé pour la fabrication d'une composition pour le soin oral selon l'une quelconque des revendications précédentes comprenant les étapes de :
i) mélange du matériau de premier constituant avec un solvant pour produire une suspension ;
ii) chauffage de la suspension à de 25°C à 95°C pendant de 0,5 heure à 3 heures ;
iii) addition de réactif approprié pour production *in situ* du matériau de second constituant dans la suspension pour former un mélange réactionnel ;
iv) récupération du matériau composite à partir du mélange réactionnel ; et
v) combinaison du matériau composite avec un support physiologiquement acceptable.

14. Procédé selon revendication 13, dans lequel le réactif approprié pour une portion anionique du matériau de second constituant comprend du silicate de sodium, silicate de potassium, alginate de sodium, sulfate de sodium, orthosilicate de tétraéthyle ou des mélanges de ceux-ci et dans lequel le réactif approprié pour une portion cationique du matériau de second constituant comprend du gluconate de calcium, oxyde de calcium, lactate de calcium, acétate de calcium, hydroxyde de calcium, sulfate de calcium, calcium carboxyméthylcellulose, chlorure de calcium, chlorure de potassium, chlorure de magnésium, chlorure de strontium, chlorure d'aluminium ou des mélanges de ceux-ci.

15. Procédé pour le blanchiment des dents d'un individu comprenant l'application de la composition selon l'une quelconque des revendications 1 à 12 sur au moins une surface des dents de l'individu.

16. Composition pour le soin oral selon l'une quelconque des revendications 1 à 12 précédentes ou composition pour le soin oral pouvant être obtenue par un procédé selon la revendication 13 ou 14 pour une utilisation comme un médicament.

17. Composition pour le soin oral selon l'une quelconque des revendications 1 à 12 précédentes ou pouvant être obtenue par un procédé selon la revendication 13 ou 14 pour une utilisation dans la réduction de la sensibilité dentaire.

18. Composition pour le soin oral selon l'une quelconque des revendications 1 à 12 précédentes ou pouvant être obtenue par un procédé selon la revendication 13 ou 14 pour une utilisation dans la reminéralisation des dents d'un individu comprenant l'étape d'application de la composition sur au moins une des dents de l'individu.
